Europäisches Patentamt

European Patent Office

Office européen des brevets

(11) Publication number: **0 255 383 B1**

(12) **EUROPEAN PATENT SPECIFICATION**

(45) Date of publication of patent specification: **22.04.92**  (51) Int. Cl.5: **C12N 7/01, C12N 15/39**

(21) Application number: **87306754.0**

(22) Date of filing: **30.07.87**

The file contains technical information submitted after the application was filed and not included in this specification

(54) **Improved mutant vaccinia virus and process for production thereof.**

(30) Priority: **31.07.86 JP 178924/86**

(43) Date of publication of application:
**03.02.88 Bulletin 88/05**

(45) Publication of the grant of the patent:
**22.04.92 Bulletin 92/17**

(84) Designated Contracting States:
**CH DE FR GB IT LI SE**

(56) References cited:
**EP-A- 0 110 385**
**EP-A- 0 157 528**

**PROCEEDINGS OF THE NATIONAL ACADEMY OF SCIENCES OF THE USA, vol. 82, May 1985, pages 3365-3369; E. PAEZ et al.: "Generation of a dominant 8-MDa deletion at the left terminus of vaccinia virus DNA"**

**NATURE, vol. 317, 31st October 1985, pages 813-815; R.M.L. BULLER et al.: "Decreased virulence of recombinant vaccinia virus expression vectors is associated with a thymidine kinase-negative phenotype"**

(73) Proprietor: **TOA NENRYO KOGYO KABUSHIKI KAISHA**
**1-1 Hitotsubashi, 1-Chome Chiyoda-Ku Tokyo 100(JP)**

Proprietor: **CHIBA PREFECTURE**
**1-1, Ichiba-cho**
**Chiba-shi Chiba(JP)**

(72) Inventor: **Sugimoto, Masanobu**
**2-3-4-702, Tate**
**Shiki-shi Saitama(JP)**
Inventor: **Nishimaki, Fukumi**
**Takayama Manshion 309 1-17-10, Nishitsurugaoka**
**Ooimachi Iruma-gun Saitama(JP)**
Inventor: **Muruyama, Tadashi**
**5-6-13, Higashimachi**
**Koganei-shi Tokyo(JP)**
Inventor: **Miki, Keizaburo**
**1-35-16, Shimizu Suginami-ku Tokyo(JP)**
Inventor: **Morita, Michio**
**1-10-4, Chishirodai higashi**
**Chiba-shi Chiba(JP)**

BIOLOGICAL ABSTRACTS, vol. 80, 1985, abstract no. 67262, Philadelphia,,P.A., US; M. SUGIMOTO et al.: "Gene structures of low-neurovirulent vaccinia virus LC16m0, LC16m8 and their Lister original strains", MICROBIOL. IMMUNOL. 29(5), 421-428

BIOLOGICAL ABSTRACTS, vol. 83, 1987, abstract no. 107689, Philadelphia, P.A., US; M. MORITA et al.: "Recombinant vaccinia virus LC16m0 or LC16m8 that expresses hepatitis B surface antigen while preserving the attenuation of the parental virus strain", & VACCINE 5(1), 65-70, 1987

Inventor: **Suzuki, Kazuyoshi**
**3-11-2, Kounodai**
**Ichikawa-shi Chiba(JP)**

(74) Representative: **Baverstock, Michael George**
**Douglas et al**
**BOULT, WADE & TENNANT 27 Furnival Street**
**London, EC4A 1PO(GB)**

**Description**

The present invention relates to an improved mutant vaccinia virus and a process for the production thereof.

The vaccinia virus was originally developed as a vaccination virus. Recently, however, vaccinia virus has drawn attention as a vector for a recombinant vaccinia virus. In the recombinant vaccinia virus technique, a gene coding for an antigenic protein of a particular pathogenic virus, i.e., exogenous antigen gene, is introduced into a vaccinia virus gene as a vector for the construction of a recombinant vaccinia virus. According to this technique, the development of a vaccine against viral disease, which has been difficult so far, can be realized. Therefore, the vaccinia virus has become useful as a vector virus for the construction of a recombinant vaccinia virus.

A virus for a vaccine must have both a high proliferation potency and a low toxicity (pathogenic potency?), especially neurovirulence. Therefore, to obtain a recombinant vaccinia virus by introducing an exogenous antigen gene into a vector vaccinia virus, the vector vaccinia virus per se. must have the above-mentioned desired properties.

The Lister original (LO) strain of the vaccination virus has a high proliferation potency, but at the same time a relatively high neurovirulence, which is disadvantageous as the vaccination virus and as the vector for the construction of a recombinant virus as a vaccine. As an improved vaccination virus which does not have this disadvantage, a mutant Lister clone l6 (LCl6) has been from the Lister original (LO) strain (Hashisum et al. Vacinia Viruses as Vectors for Vaccine Antigens, Elsevier, l985, p 87-99).

The above-mentioned LCl6 was passaged six times on RK cells and cloned by a plaque method to obtain a mutant LCl6m0 strain which provides relatively small and even-size pocks on a chorio-allantoic membrane. The mutant LCl6m0 was further passaged three times and recloned to isolate an LCl6m8 strain, which exhibits a very small pock size. The LCl6m8 is advantageous as a vaccination virus in that it has a low neurovirulence, a low proliferation potency in the brain, a low invasion potency, a low recovery of virus from virus inoculated brain, and provides a good histopathological feature of the brain after inoculation of virus. However, the LCl6m8 is disadvantageous in that it has a low proliferation potency in the skin and a low in vitro proliferation on Vero cells (Hashizume, Rinsho To Virus, Vol 3, No. 3,225-235, l975).

The gene structures of the original LO strain and the mutant LCl6m8 strain were compared, and it was found that a Hind III D fragment of about l0 kb derived from the LCl6m8 carries an Xho I site, although a corresponding fragment derived from the LO does not carry the Hind III site, suggesting that gene related to the proliferation and neurotoxicity of the virus are present on the Hind III D fragment (Sugimoto et al., Microbiol. Immunol. Vol 29 (5), 40l-428, l985).

As seen from above, although a vaccinia virus which has a high proliferation potency and high pathogenic properties, and a vaccinia virus which has a low proliferation potency and low pathogenic properties, including neurotoxicity, are known, a vaccinia virus possessing a high proliferation potency with low pathogenic properties is not known.

The present invention provides a vaccinia virus which has both a high proliferation potency and low pathogenic properties, especially a low neurovirulence, and therefore, is useful as a vector for the construction of various recombinant virus vaccines.

More specifically, the present invention provides an improved mutant vaccinia virus comprising a parent mutant vaccinia virus of LC16 series derived from a vaccinia virus Lister original wherein at least a part of a Hind III D fragment region of the parent mutant vaccinia virus gene wherein the Hind III D fragment region is of about 10kb and carries an Xho I site has been replaced by at least a corresponding part of a Hind III D fragment of the vaccinia virus Lister original gene, wherein the parent mutant vaccinia virus provides a pock size and plaque size on RK13 cells smaller than those provided by the Lister Original, has a proliferation potency on YTV cells lower than that of the Lister original, and has a neurovirulence, assessed by recovery of an intrabrain virus, lower than that of the Lister original, and wherein the improved mutant vaccinia virus provides a pock size and plaque size on RK13 cells that is approximately the same as those of the Lister original, has a proliferation potency on YTV cells that is approximately the same as that of Lister original, and has a neurovirulence, assessed by a recovery of an intrabrain virus, lower than that of the Lister original.

The present invention also provides a process for the production of an improved mutant vaccinia virus as defined above, comprising the steps of:

preparing a parent mutant vaccinia virus of LC16 series derived from a vaccinia virus Lister original, wherein the parent mutant vaccinia virus provides a pock size and plaque size on RK13 cells that is smaller than those provided by the Lister original, has a proliferation potency on YTV cells that is lower than that of the Lister original, and has a neurovirulence, assessed by a recovery of an intrabrain virus, lower than that of the Lister original;

preparing a Hind III D fragment from the Lister original gene;

replacing at least a part of the Hind III D fragment region of the parent mutant vaccinia virus gene wherein the Hind III D fragment region is of about 10kb and carries an Xho I site, by at least a part of the Hind III D fragment of the Lister original gene; and

selecting an improved mutant vaccinia virus providing a pock size and plaque size on RK13 cells that is approximately the same as those of the Lister original, having a proliferation potency on YTV cells that is approximately the same as that of Lister original, and having a neurovirulence, assessed by a recovery of an intrabrain virus, lower than that of the Lister original.

In the drawings:

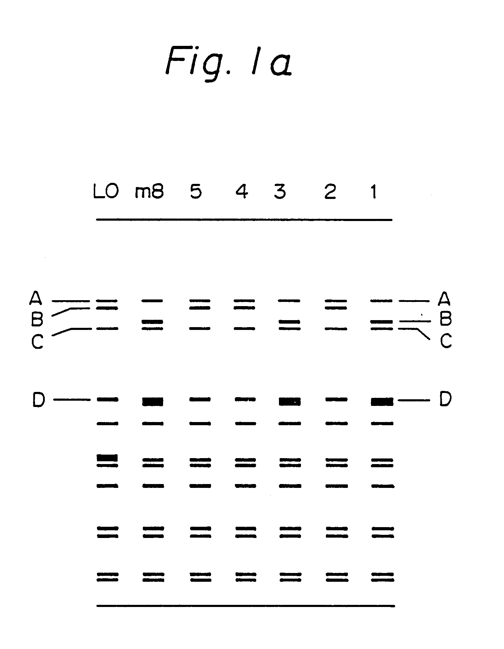

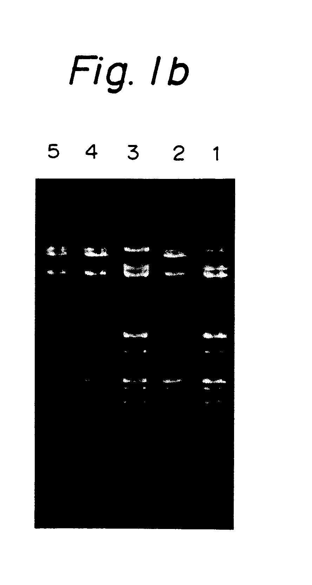

Figure 1a is a sketch of electrophoresis patterns of Xho I digestion fragments derived from genes of parent vaccinia virus strains LO and mutant LO16m8, as well as mutant strains LOTC-1, LOTC-2, LOTC-3, LOTC-4 and LOTC-5 of the present invention. In these digestion patterns, digestion fragments from each strain are designated as A, B, C, D ... according to the size of each fragment; and Fig. 1b is a photograph corresponding to a part of the sketch of Fig. 1a;

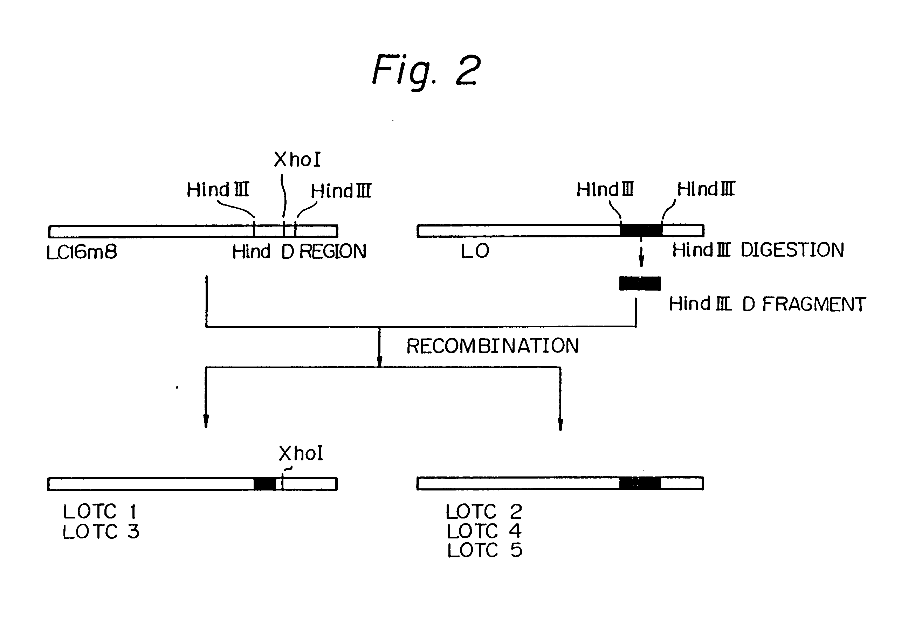

Fig. 2 schematically represents a process of recombination between a gene fragment derived from a parent LO strain and a gene fragment of a parent mutant LC16m8 strain to form a recombinant gene, wherein a Hind III D fragment of the LC16m8 strain genome is replaced with a corresponding fragment of the LO strain; and

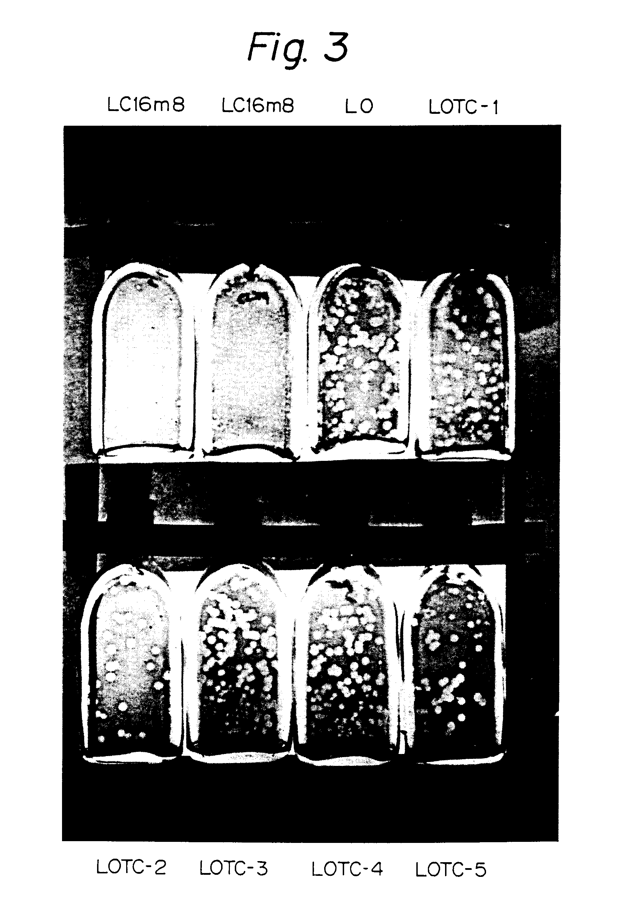

Fig. 3 is a photograph comparing the size of plaques of the parent strains LO and LC16m8 as well as the LOTC-1 to LOTC-5 of the present invention.

An improved mutant vaccinia virus of the present invention can be obtained by replacing a part of or the entire Hind III D region in a parent mutant virus with a corresponding Hind III D region derived from an LO viral gene.

(1) Parent mutant strain

The parent mutant virus is a viral strain which is used as a parent, i.e., a starting virus, for the construction of an improved mutant virus of the present invention, but is a mutant derived from an original strain LO. These parent mutant viruses have a low proliferation potency and low pathogenic properties, and are exemplified by an LCl6 series strains. The process for the construction and the properties of these parent mutant viruses are disclosed in detail in Rinsho To Virus, Vol 3, No. 3, 229-235, 19875. These mutant viruses are available from Chiba Serum Institute, Japan. The properties of the preferable parent mutant LCl6m8 are compared with those of the original LO in Table I.

## Table 1

| Virus strain | LO | LC16m8 |
|---|---|---|
| Temperature at which virus cannot proliferate on RK cells | >41°C | 40.5°C |
| Plaque size on RK cells | Large | Medium |
| Pock size | Large | Small |
| Proliferation potency on | | |
| CAM | +++ | +++ |
| Vero cells | +++ | + |
| RK cells | +++ | +++ |
| CEF cells | ++ | ++ |
| Neurovirulence to central nervous system | | |
| Proliferation | | |
| Rabbit | ++ | ± |
| Monkey | +++ | + |
| Mouse | + | |
| Invasion | | |
| Mouse | +++ | − |
| Proliferation in skin | | |
| Rabbit | +++ | + |
| Human | ++ | + |
| Antibody productivity | | |
| Rabbit | ++ | ++ |
| Human | ++ | ++ |

(2) Construction of improved mutant virus strains

The construction process of the improved mutant virus strains of the present invention is described with the use of an LCl6m8 strain as a parent mutant virus.

5

First, a Hind III D fragment of the LO virus DNA was prepared as follows: virion of the LO strain were prepared by as follows: Viruses were proliferated in RKl3 cells. After freezing and thawing of the cultured cells, cell debris was removed by centrifugation. Virion were collected by the ultracentrifugation of the supernatant. Virion were then purified by sucrose density ultracentrifugation. The virion were treated with a phosphate buffer (pH 6.8) containing l% of a surfactant Sarkosyl NL97 and 6M of urea to extract the DNA according to Juklik, W.K., Biochem. Biophys. Acta, 6l, 290-30l, l962. The DNA preparation was extracted with phenol/chloroform to eliminate proteins, and the purified DNA was digested with l0 units of Sma I in l0 $\mu$l of a buffer containing l0 mM Tris-HCl (pH 7.5), 7 mM $MgCl_2$, 20 mM KCl, 7 mM 2-mercaptoethanol and l00 $\mu$g/ml bovine serum albumin at 30°C for l hour. As a result the DNA was cleaved into a large fragment A and a small fragment B. The fragment B (l $\mu$g) was further digested with l0 units of Hind III in l0 $\mu$l of a buffer containing l0 mM Tris-HCl (pH 8.0), 7 mM $MgCl_2$, l0 mM NaCl and l00 $\mu$g/ml bovine serum albumin at 37°C for l hour. The resulting fragments were cloned in plasmid pUC9 as follows.

The Hind III digests were extracted with phenol followed by phenol-chloroform-isoamylalcohol, and precipitated with ethanol. Recovered DNA was ligated with l0 units of T4 DNA ligase in 50 mM Tris-HCl (pH 7.6), 6.6 mM $MgCl_2$, l0 mM dithiothreitol, 66 $\mu$M ATP into pUC9 which had been digested with Hind III. E. coli (JMl09) was transformed with the ligated plasmid. White colonies containing the plasmid with foreign gone were selected. A clone containing a plasmid containing a DNA fragment of about l0 kb was selected as follows. DNA was extracted from the transformed E. coli (JMl09) according to boiling method (Molecular Cloning, T. Maniatis et al., Cold Spring Harbor Laboratory, l982, pp 366-369. DNA was digested with a restriction enzyme Hind III, and the resultant DNA fragment was separated by agarose electropholesis. The plasmids producing about l0 kb flagment were cloned. It was confirmed by the Southern blotting method that the DNA fragment of about l0 kb was a Hind D III fragment. That is, this DNA fragment migrated to the same position as that of the Hind III D fragment prepared by digesting an LO virus gene directly with the Hind III in electrophoresis and hybridized with an Xho B fragment (Sugimoto et al., Microbiol. Immunol. 29, 420, l985).

The Hind III D fragment thus obtained from the LO virus gene was transfected to the LCl6m8 virus gene as follows: RKl3 cells were inoculated into a culture bottle containing 5 ml of RPMl640 containing l0% fetal bovine serum, having a culture surface of 25 $cm^2$ to form a monolayer, and a $10^5$ PFU/bottle (about 0.l PFU/cell) of the LCl6m8 virus was infected to the cultured cells. Next, the above-mentioned Hind III D fragment preparation derived from the LO strain was added to the infected monolayer by the calcium phosphate method. 2 $\mu$g of the plasmid containing the Hind III D fragment with 20 $\mu$g of calf-thymus DNA as a carrier in 0.24 M $CaCl_2$ solution (2.5 ml) was mixed by drop with bubbling of air to the solution containing 2.5 ml of 2 $\times$ HBS (l0 g HEPES and 6 g NaCl/l, pH 7.l0) and 0.05 ml of l00 $PO_4$ (70 mM $NaH_2PO_4$ and 70 mM $NaHPO_4$). The solution was stood for 30 min. The solution with precipitated DNA with calcium phosphate was added to RK l3 cell culture infected with LCl6m8. After six hours, the culture medium was exchanged and culturing was carried out for 48 hours at 37°C. The cultured broth was subjected to repeated freezing and thawing to recover a virus, and a 5 ml/bottle of virus solution was obtained. The virus in this solution was then titrated.

Next, viruses subjected to the recombination treatment described above were screened to select virus strains forming a large plaque, as follows: RKl3 cells were inoculated into a plastic petri dish having a diameter of 6 cm and containing 5 ml of PBRl640 medium with l0% fetal bovine serum, and cultured for 24 hours at 37°C to form a monolayer. 0.l ml of l/l0 to l/l00 - dibuted above-prepared virus solution was added to the monolayer, and the cultured cells adsorbed the virus. 3 ml of Eagle's MEM medium containing l% agar and l0% bovine serum was layered over the monolayer according to a conventional method, and culturing was carried out for three days at 37°C. In this manner, a virus which forms large plaques having a diameter of about 3 to 4 mm was obtained. The above-mentioned cloning procedure was repeated three times to clone the desired virus strains.

These virus strains formed large plaques generated at a ratio of 4 to 8 per l00PFU of starting virus. Since the LCl6m8 strain does not form large plaque on RKl3 cells and there is little possibility of the occurrence of a spontaneous mutation providing mutants at a high ratio as described above, it is believed that most of the virus strains forming large plaques are generated by a recombination between the Hind III D regions of the LO virus gene and the LCl6m8 virus gene.

Five typical virus strains, i.e., LOTC-l, LOTC-2, LOTC-3, LOTC-4, and LOTC-5, were selected, cloned and further characterized.

(3) DNA analysis

DNAs were extracted from the LO strain, LCl6m8 strain, and the above-mentioned five strains of the present invention according to a conventional method, digested with a restriction enzyme Xho I, and the digestion product was analyzed by agarose gel electrophoresis. The separation patterns are shown in Figs. la and lb.

As seen from Fig. I, the size of the B fragment of the LCl6m8 strain is smaller than that of the B fragment of the LO strain. However, the LCl6m8 strain provides an extra D fragment which is not derived from the LO strain. This means that the Hind III D fragment contains an XhoI site which is not present at the corresponding site of the LO virus gene. Among the improved mutant strains of the present invention, LOTC-2, LOTC-4 and LOTC-5 provide the same digestion pattern as that of the LO strain, revealing that the recombination occurred at region encompassing the Xho I site in question. However, the LOTC-I and LOTC-3 strains provide the same digestion pattern as that of the LCl6m8 strain, revealing that the recombination occurs at an region other than the Xho I site.

Taking the above-mentioned result into consideration, the process of the recombination is schematically shown in Fig. 2.

(4) Characterization of improved mutant virus strains

The properties of the LOTC series mutant strains are summarized in comparison with the LO and LCl6m8 strains, as follows.

a) Neurovirulence

Each of the above-mentioned virus was inoculated into the brain of a rabbit at an amount of $10^{6.8}$ of $TCID_{50}$, and six days later, the rabbit was killed and the neurovirulence was assessed. A WR strain ( + + ), LO strain ( + ), LCl6m0 strain (±), and LCl6m8 (-) strains were used as controls. The results showed that the LOTC-3 and LOTC-5 strains were no less toxic than the LCl6m8, and the LOTC-I, LOTC-2 and LOTC-4 strains were less toxic than the LCl6m0. These results are shown in Table 2.

Table 2

| Virus strain | Recovery at sixth day ($\log_{10}TCID_{50}$/ml) | Assessment by recovery |
|---|---|---|
| LOTC-1 | <0.5, 2.5 | ± |
| LOTC-2 | <0.5, 1.25, 1.75, 2.0 | + |
| LOTC-3 | <0.5, <0.5, <0.5, <0.5 | - |
| LOTC-4 | <0.5, 3.0 | ± |
| LOTC-5 | <0.5, <0.5 | - |
| LO | 2.5, 3.5, 3.75, 4.75 | + + |
| SC16m8 | <0.5, <0.5, <0.5 | - |
| LC16m0 | <0.5, <0.5, 4.25 | + |
| WR | 5.75, 6.5, 7.0 | + + + |

b) In vitro virus proliferation

i) Plaque size on RK13 cells

As shown in Fig. 3, plaques of the LO strain are very much larger than those of the LC16m8, and the plaques of the LOTC-1 to LOTC-5 strains are similar in size to those of the LO strain.

ii) Proliferation on YTV (Vero cells)

Although the LC16m8 strain makes small plaques but can proliferate on RK13 cells, this LC16m8 strain has a very poor proliferation on YTV cells. Accordingly, the RK13/YTV ratio in plaque number is as high as about 500. Conversely, the LO strain is highly proliferate on YTV cells, and accordingly, the RK13/YTV ratio is less than 1. The RK13/YTV ratios of the LOTC-1 to LOTC-5 strains of the present invention are similar to that of the LO strain. The results are shown in Table 3.

iii) Pock size

7

The pock size of the LC16m8 strain observed on a fertilized hen's egg is small, and that of the LO strain is large. These pock sizes of the LOTC series strains of the present invention were medium to large, and those of the LOTC strains not having the Xho I site in the recombination region in question were larger than those of the LOTC strains having the Xho I site. The results are shown in Table 3.

Table 3

| Virus strain | Proliferation property RK13/YTV | Plaque size on RK13 cells | Pock size |
|---|---|---|---|
| LO | 0.22 | Large | Large |
| LC16m8 | 500 | Small | Small |
| LOTC-1 | 0.91 | Large | Medium |
| LOTC-2 | 0.68 | Large | Large |
| LOTC-3 | 0.71 | Large | Large |
| LOTC-4 | 0.68 | Large | Large |
| LOTC-5 | 1.15 | Large | Large |

As seen from the above-mentioned characteristics of the mutant virus strains of the present invention, the present invention provides improved mutant virus strains and a process for the production thereof. The present mutant virus strains have a proliferation potency as high as that of the LO strain, and a neurovirulence lower than that of the LO strain.

These mutant virus strains are promising as attenuated vaccination viruses as well as vectors for the construction of a recombinant vaccinia virus.

**Claims**

1. A mutant vaccinia virus comprising a parent mutant vaccinia virus of LC16 series derived from a vaccinia virus Lister original wherein at least a part of the Hind III D fragment region of the parent mutant vaccinia virus gene, wherein the Hind III D fragment region is of about 10kb and carries an Xho I site, has been replaced by at least a corresponding part of the Hind III D fragment of the vaccinia virus Lister original gene, wherein the parent mutant vaccinia virus provides a pock size and plaque size on RK13 cells smaller than those provided by the Lister Original, has a proliferation potency on YTV cells lower than that of the Lister original, and has a neurovirulence, assessed by recovery of an intrabrain virus, lower than that of the Lister original, and wherein the improved mutant vaccinia virus provides a pock size and plaque size on RK13 cells that is approximately the same as those of the Lister original, has a proliferation potency on YTV cells that is approximately the same as that of Lister original, and has a neurovirulence, assessed by a recovery of an intrabrain virus, lower than that of the Lister original.

2. An improved mutant vaccinia virus according to claim 1 wherein the parent mutant vaccinia virus is an LC16m8 strain derived from the Lister original via passages.

3. An improved mutant vaccinia virus according to claim 1 wherein at least a part of the Hind III D fragment region of the parent mutant vaccinia virus gene encompassing an Xho I site has been replaced by at least a corresponding part of a Hind III D fragment of the Lister original gene.

4. An improved mutant vaccinia virus according to claim 1, wherein a part of the Hind III D fragment region of the parent mutant vaccinia virus gene excluding the Xho I site has been replaced by at least a corresponding part of the Hind III D fragment of the Lister original gene.

5. A process for the production of an improved mutant vaccinia virus as defined above, comprising the steps of:
    preparing a parent mutant vaccinia virus of LC16 series derived from a vaccinia virus Lister original, wherein the parent mutant vaccinia virus provides a pock size and plaque size on RK13 cells that is smaller than those provided by the Lister original, has a proliferation potency on YTV cells that is lower than that of the Lister original, and has a neurovirulence, assessed by a recovery of an intrabrain virus, lower than that of the Lister original;
    preparing a Hind III D fragment from the Lister original gene;

8

replacing at least a part of the Hind III D fragment region of the parent mutant vaccinia virus gene wherein the Hind III D fragment region is of about 10kb and carries an Xho I site, by at least a corresponding part of the Hind III D fragment of the Lister original gene; and

selecting an improved mutant vaccinia virus providing a pock size and plaque size on RK13 cells that is approximately the same as those of the Lister original, having a proliferation potency on YTV cells that is approximately the same as that of Lister original, and having a neurovirulence, assessed by a recovery of an intrabrain virus, lower than that of the Lister original.

6. A process according to claim 5, wherein the replacement is carried out by a recombination in animal cells.

**Revendications**

1. Un virus mutant de la vaccine comprenant un virus mutant parent de la vaccine de la série LC16 dérivé d'une lignée originale de Lister du virus de la vaccine, dans lequel au moins une partie de la zone du fragment Hind III D du gène du virus mutant parent de la vaccine, dans laquelle la zone du fragment Hind III D est d'environ 10 kb et porte un site Xho I, a été remplacée par au moins une partie correspondante du fragment Hind III D du gène de la lignée originale de Lister du virus de la vaccine, dans lequel le virus mutant parent de la vaccine donne une grandeur de pustule et une gandeur de plaque sur des cellules RK13 inférieures à celles données par la lignée originale de Lister, a un pouvoir de prolifération sur les cellules YTV inférieur à celui de la lignée originale de Lister et a une neurovirulence, évaluée par récupération d'un virus intracérébral, inférieure à celle de la lignée originale de Lister et dans lequel le virus mutant amélioré de la vaccine donne une grandeur de pustule et une grandeur de plaque sur les cellules RK13 qui sont environ égales à celles de la lignée originale de Lister, a un pouvoir de prolifération sur les cellules YTV qui est environ égal à celui de la lignée originale de Lister et a une neurovirulence, évaluée par récupération d'un virus intracérébral, inférieure à celle de la lignée originale de Lister.

2. Un virus mutant amélioré de la vaccine suivant la revendication 1, dans lequel le virus mutant parent de la vaccine est une lignée LC16m8 dérivée de la lignée originale de Lister par des passages.

3. Un virus mutant amélioré de la vaccine suivant la revendication 1, dans lequel au moins une partie de la zone du fragment Hind III D du gène du virus mutant parent de la vaccine englobant un site Xho I a été remplacée par au moins une partie correspondante d'un fragment Hind III D du gène de la lignée originale de Lister.

4. Un virus mutant amélioré de la vaccine suivant la revendication 1, dans lequel une partie de la zone du fragment Hind III D du gène du virus mutant parent de la vaccine excluant le site Xho I a été remplacée par au moins une partie correspondante du fragment Hind III D du gène de la lignée originale de Lister.

5. Un procédé pour la production d'un virus mutant amélioré de la vaccine tel que décrit ci-dessus, comprenant les étapes consistant à:

préparer un virus mutant parent de la vaccine de la série LC16 dérivée d'une lignée originale de Lister du virus de la vaccine, dans lequel le virus mutant parent de la vaccine donne une grandeur de pustule et une gandeur de plaque sur des cellules RK13 inférieures à celles données par la lignée original de Lister, a un pouvoir de prolifération sur les cellules YTV inférieur à celui de la lignée originael de Lister et a une neurovirulence, évaluée par récupération d'un virus intracérébral, inférieure à celle de la lignée originale de Lister,

préparer un fragment Hind III D à partir d'un gène de la lignée originale de Lister,

remplacer au moins une partie de la zone du fragment Hind III D, dans laquelle la zone du fragment Hind III D est d'environ 10 kb et porte un site Xho I, par au moins une partie correspondante du fragment Hind III D du gène de la lignée originale de Lister, et

sélectionner un virus mutant amélioré de la vaccine donnant une grandeur de pustule et une gandeur de plaque sur les cellules RK13 qui sont environ égales à celles de la lignée originale de Lister, ayant un pouvoir de prolifération sur les cellules YTV qui est environ égal à celui de la lignée originale de Lister et ayant une neurovirulence, estimée par une récupération d'un virus intracérébral, inférieure à celle de la lignée originale de Lister.

**6.** Un procédé suivant la revendication 5, dans lequel le remplacement est effectué par une recombinaison dans des cellules animales.

**Patentansprüche**

**1.** Ein Mutanten-Vacciniavirus, umfassend ein elterliches Mutanten-Vacciniavirus der LC16 Reihen, das abgeleitet von einem Lister-Original-Vacciniavirus ist, bei dem wenigstens ein Teil des Hind III D Fragmentbereichs des elterlichen Mutanten-Vacciniavirusgens, wobei der Hind III D Fragmentbereich etwa 10kb lang ist und eine Xho I Stelle enthält, durch wenigstens einen entsprechenden Teil des Hind III D Fragments des Lister-Original-Vacciniavirusgens ersetzt ist, wobei das elterliche Mutanten-Vacciniavirus eine Pock-Größe und eine Plaquegröße bei RK13-Zellen schafft, die kleiner sind als diejenigen, die durch das Lister Original geschaffen werden, das eine Proliferationsfähigkeit bei YTV-Zellen aufweist, die niedriger ist als diejenige vom Lister Original und das eine Neurovirulenz aufweist, die niedriger ist als diejenige vom Lister Original, abgeschätzt über die Wiedergewinnung eines gehirninternen Virus, und bei dem das verbesserte Mutanten-Vacciniavirus eine Pock-Größe und Plaquegröße bei RK13-Zellen schafft, die in etwa diegleichen sind wie diejenigen vom Lister Original, das eine Proliferationsfähigkeit bei YTV-Zellen aufweist, die in etwa diegleiche ist wie diejenige vom Lister Original und das eine Neurovirulenz aufweist, die niedriger ist als diejenige vom Lister Original, abgeschätzt über die Wiedergewinnung eines gehirninternen Virus.

**2.** Ein verbessertes Mutanten-Vacciniavirus nach Anspruch 1, wobei das elterliche Mutanten-Vacciniavirus ein LC16m8 Stamm ist, der vom Lister Original durch Passagen abgeleitet ist.

**3.** Ein verbessertes Mutanten-Vacciniavirus nach Anspruch 1, bei dem wenigstens ein Teil des Hind III D Fragmentbereichs des elterlichen Mutanten-Vacciniavirusgens, das eine Xho I Stelle enthält, durch wenigstens einen entsprechenden Teil eines Hind III D Fragments des Lister-Original-Gens ersetzt ist.

**4.** Ein verbesserte Mutanten-Vacciniavirus nach Anspruch 1, bei dem ein Teil des Hind III D Fragmentbereichs des elterlichen Mutanten-Vacciniavirusgens unter Ausschluß der Xho I Stelle durch wenigstens einen entsprechenden Teil des Hind III D Fragments des Lister-Original-Gens ersetzt ist.

**5.** Ein Verfahren zur Herstellung eines wie oben definierten verbesserten Mutanten-Vacciniavirus, die folgenden Schritte umfassend:
Herstellen eines elterlichen Mutanten-Vacciniavirus der LC16 Reihen, das abgeleitet ist aus einem Lister-Original-Vacciniavirus, wobei das elterliche MutantenVacciniavirus eine Pock-Größe und eine Plaquegröße bei RK13-Zellen schafft, die kleiner ist als diejenigen, die durch das Lister Original geschaffen werden, das eine Proliferationsfähigkeit bei YTV-Zellen aufweist, die niedriger ist als diejenige vom Lister Original und das eine Neurovirulenz aufweist, die niedriger ist als diejenige vom Lister Original, abgeschätzt über die Wiedergewinnung eines gehirninternen Virus;
Herstellen eines Hind III D Fragments aus dem Lister-Original-Gen;
Ersetzen von wenigstens einem Teil des Hind III D Fragmentbereichs des elterlichen Mutanten-Vacciniavirusgens, bei dem der Hind III D Fragmentbereich etwa 10 kb lang ist und eine Xho I Stelle enthält, durch wenigstens einen entsprechenden Teil des Hind III D Fragments des Lister-Original-Gens; und
Auswählen eines verbesserten Mutanten-Vacciniavirus, das eine Pock-Größe und eine Plaquegröße bei RK13-Zellen schafft, die in etwa diegleichen sind wie diejenigen vom Lister Original, das eine Proliferationsfähigkeit bei YTV-Zellen hat, die in etwa diegleiche ist wie diejenige vom Lister Original und das eine Neurovirulenz aufweist, die niedriger ist als diejenige vom Lister Original, abgeschätzt über die Wiedergewinnung eines gehirninternen Virus.

**6.** Ein Verfahren nach Anspruch 5, wobei das Ersetzen durch eine Rekombination in tierischen Zellen durchgeführt wird.

# Fig. 1a

# Fig. 1b

# Fig. 2

LC16m8 — HindⅢ, XhoI, HindⅢ — Hind D REGION

LO — HindⅢ, HindⅢ — HindⅢ DIGESTION

HindⅢ D FRAGMENT

RECOMBINATION

LOTC 1
LOTC 3 — XhoI

LOTC 2
LOTC 4
LOTC 5

EP 0 255 383 B1

# *Fig. 3*

LC16m8    LC16m8    LO    LOTC-1

LOTC-2    LOTC-3    LOTC-4    LOTC-5